# EUROPEAN PATENT APPLICATION

(11) **EP 1 477 113 A1**
(43) Date of publication of application: **17.11.2004**
(21) Application number: 04009006.0
(22) Date of filing: 15.04.2004
(51) Int. Cl.: A61B 5/03, A61B 5/028, G01F 1/684

(54) **Flow sensor device for endoscopic third ventriculostomy**

(30) Priority: 24.04.2003 US 423144
(71) Applicant: Medtronic Vascular, Inc., Santa Rosa, California 95403 (US)
(72) Inventor: Stiger, Mark L., Windsor California 95492 (US); Carney, James K., Eden Praire Minnesota 55347 (US); Kaemmerer, William F., Edina Minnesota 55435 (US); Yamasaki, Sonny, Rohnert Park California 94928 (US)
(74) Representative: Zimmermann, Gerd Heinrich

(57) **Abstract**

An ETV procedure includes forming a burr hole in a human skull; passing an endoscopic third ventriculostomy (ETV) probe (102) through the burr hole; fenestrating the floor of the third ventricle with the ETV probe (102) to form an opening in the floor; and measuring a flow of cerebrospinal fluid (CSF) with a flow sensor of the ETV probe. The ETV procedure further includes deploying a membrane eyelet (402) into the opening; and measuring a flow of CSF through the opening with a flow sensor (412) of the membrane eyelet (402). An improved ETV probe (102) and membrane eyelet (402) are provided.

## Description

### BACKGROUND OF THE INVENTION

### Field Of The Invention

The present invention relates to medical devices. More particularly, the present invention relates to devices for forming, maintaining, and verifying the patency of an opening or orifice in a septum (or membrane).

### Description Of The Related Art

Noncommunicating hydrocephalus is a condition that results in the enlargement of the ventricles caused by abnormal accumulation of cerebrospinal fluid (CSF) within the cerebral ventricular system.

In noncommunicating hydrocephalus, there is an obstruction at some point in the ventricular system. The cause of noncommunicating hydrocephalus usually is a congenital abnormality, such as stenosis of the aqueduct of Sylvius, congenital atresia of the foramina of the fourth ventricle, or spina bifida cystica. There are also acquired versions of hydrocephalus that are caused by a number of factors including subarachnoid or intraventricular hemorrhages, infections, inflammation, tumors, and cysts.

The main treatment for hydrocephalus is venticuloperitoneal (VP) shunts. The VP shunts are catheters that are surgically lowered through the skull and brain. The VP shunts are then positioned in the lateral ventricle. The distal end of the catheter is tunneled under the skin and positioned in the peritoneal cavity of the abdomen, where the CSF is absorbed.

However, the VP shunts have an extremely high failure rate, e.g., in the range of 30 to 40 percent. Failure includes clogging of the catheter, infection, displacement, and faulty pressure valves or one-way valves.

Another relatively newly re-introduced treatment for noncommunicating hydrocephalus is the procedure known as an endoscopic third ventriculostomy (ETV). This procedure involves forming a burr hole in the skull. A probe is passed through the burr hole, through the cerebral cortex, through the underlying white matter, and into the lateral and third ventricles. The probe is then used to poke (fenestrate) a hole in the floor of the third ventricle and underlying membrane of Lillequist.

To verify that the procedure is successful, i.e., that a hole is formed in the floor of the third ventricle and underlying membrane of Lillequist, the patient is observed with a magnetic resonance imaging (MRI) device after the probe poke. The MRI device is used to verify a flow of CSF through the hole in the floor of the third ventricle.

If the MRI device is unable to detect the flow of CSF, a determination is made that a hole in the floor of the third ventricle was not formed, and the ETV procedure is repeated.

Since the MRI device is typically located at a separate location, the ETV procedure typically requires the patient to be moved from location to location. This, in turn, increases the procedure time as well as the expense and complexity of the ETV procedure.

Further, even after successfully forming a hole in the floor of the third ventricle, the hole sometimes closes, typically within two weeks to two months after the ETV procedure. In this event, the patient will have to undergo another ETV procedure, or risk serious injury or death.

It is therefore an object of the present invention to overcome the above described disadvantages associated with the prior art at least in part.

### SUMMARY OF THE INVENTION

This object is solved by an endoscopic third ventriculostomy (ETV) probe according to claim 1 and a membrane eyelet according to claim 11. Further aspects, advantages and details of the present invention are apparent from the dependent claims, the specification and the drawings.

An ETV procedure includes forming a burr hole in a human skull; passing an endoscopic third ventriculostomy (ETV) probe through the burr hole; fenestrating the floor of the third ventricle with the ETV probe to form an opening in the floor (the underlying membrane of Lillequist is fenestrated simultaneously); and measuring or confirming a flow of cerebrospinal fluid (CSF) through the opening with a flow sensor of the ETV probe.

In the event that the flow sensor measures a flow of CSF, a determination is made that the ETV procedure is successful, i.e., that the opening in the floor was successfully formed. Accordingly, the ETV probe is withdrawn from the patient.

Thus, by using the ETV probe, the ETV procedure is determined to be successful without the necessity of transferring the patient to a separate location for observation with a magnetic resonance imaging (MRI) device as in a conventional ETV procedure. This, in turn, minimizes the ETV procedure time as well as the expense and complexity of the ETV procedure.

Further, if the flow sensor fails to detect a flow of CSF thus indicating stasis of the CSF, i.e., the flow measurement is zero, a determination is made that a hole in the floor was not formed. In this event, the physician simply makes another poke using the ETV probe to fenestrate the floor and underlying membrane.

Thus, using the ETV probe, even if the initial attempt at fenestrating the floor fails, this failure is detected immediately. The probe poke is determined to be unsuccessful without the necessity of transferring the patient to a separate location for observation with a MRI device as in a conventional ETV procedure. Further, the floor can again be poked using the same ETV probe in contrast to having to repeatedly insert and withdraw a probe as in the prior art. This, in turn, minimizes the risk of infection and eliminates the possibility of causing damage to the cerebral cortex and the underlying white matter from repeated probe insertions.

In another embodiment, an ETV procedure includes fenestrating the floor of the third ventricle with an endoscopic third ventriculostomy probe to form an opening in the floor; deploying a membrane eyelet into the opening; and measuring a flow of CSF through the opening with a flow sensor of the membrane eyelet.

In the above method, the flow of CSF through the opening, and thus the patency of the opening, is noninvasively measured and recorded.

In one embodiment, the flow sensor is a wireless flow sensor such that the flow sensor can be remotely interrogated from outside the patient. Thus, the patency of the opening can be quickly and easily verified by using the flow sensor to determining that CSF is flowing through the opening. Thus, using the membrane eyelet, the patency of the opening is verified without the necessity of observing the patient with a MRI device as in a conventional ETV procedure. This, in turn, may minimize the complexity and expense associated with the ETV procedure.

Further, using the membrane eyelet, the patency of the opening can be monitored continuously or frequently thus resulting in a rapid diagnosis of any closure of the opening. By having rapid diagnosis, the complications associated with closure of the opening can be mitigated.

### BRIEF DESCRIPTION OF THE DRAWINGS

FIG. 1 is a cross-section view of a human cranium during an endoscopic third ventriculostomy (ETV) procedure using an endoscopic third ventriculostomy probe in one embodiment according to the present invention;
FIG. 2 is an enlarged view of the region II of FIG. 1 after a fenestration has been made in a floor with the probe;
FIGS. 3, 4 and 5 are enlarged views of the region II of FIG. 1 at later stages of the ETV procedure;
FIG. 6 is a side view of a distal end of a probe similar to the probe of FIGS. 1, 2 and 3 in one embodiment according to the present invention;
FIGS. 7 and 8 are enlarged partial views of FIG. 5 of a membrane eyelet engaged with the floor after withdrawal of a dilation balloon in embodiments according to the present invention;
FIG. 9 is a circuit schematic diagram of a flow sensor of FIGS. 7 and 8;
FIG. 10 is an enlarged partial view of FIG. 5 of a membrane eyelet engaged with the floor after withdrawal of the dilation balloon in another embodiment according to the present invention; and
FIG. 11 is a circuit schematic diagram of a flow sensor of FIG. 10.

Common reference numerals are used throughout the drawings and detailed description to indicate like elements.

### DETAILED DESCRIPTION

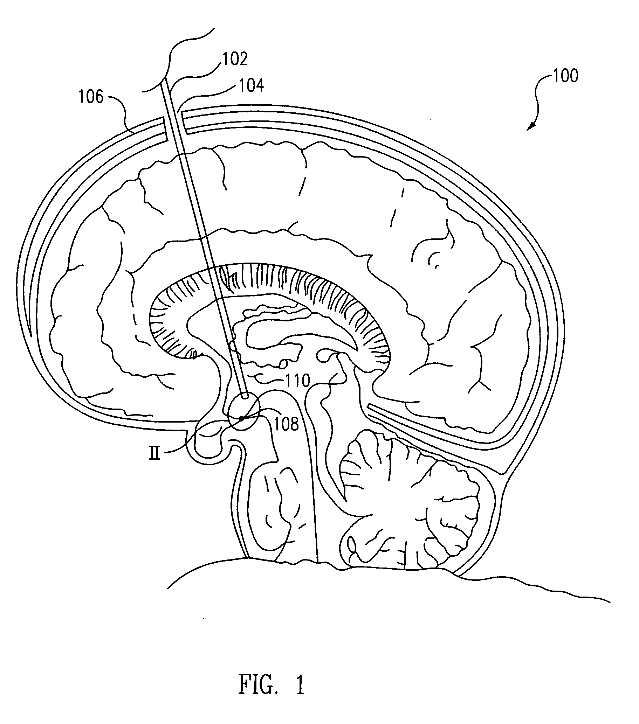

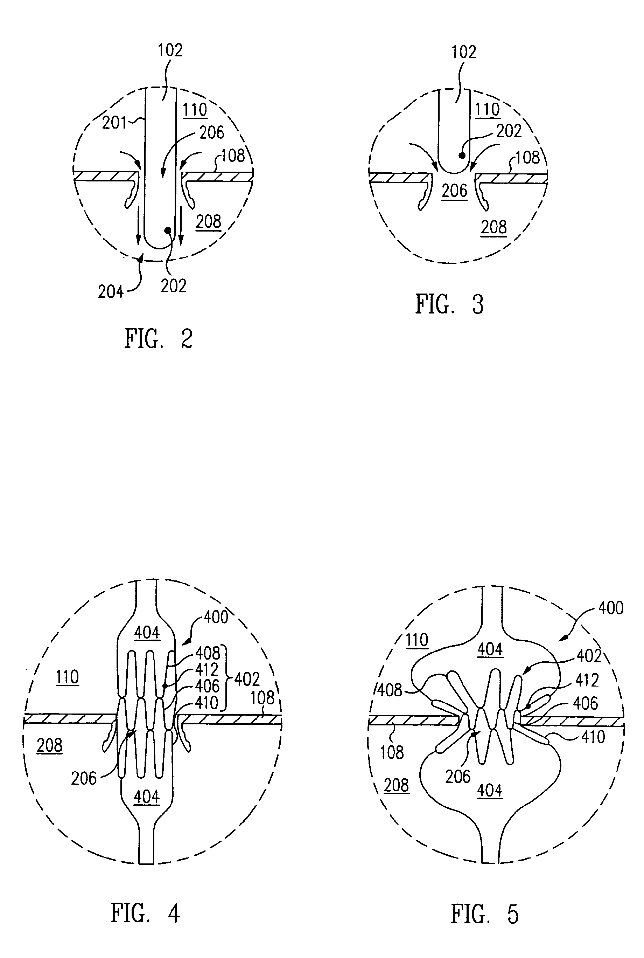

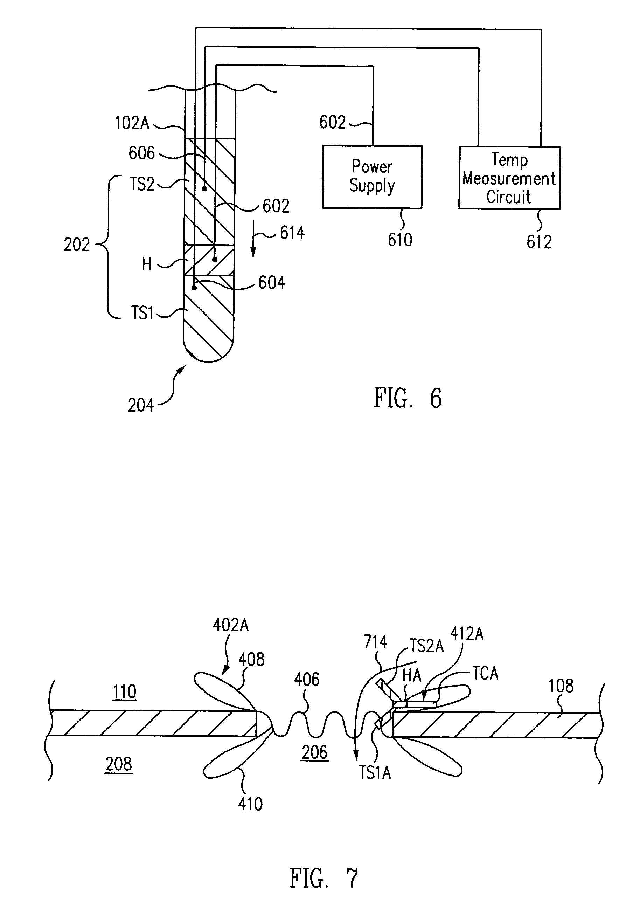

An ETV procedure includes forming a burr hole 104 (FIG. 1) in a human skull 106; passing an endoscopic third ventriculostomy (ETV) probe 102 through burr hole 104; fenestrating the floor 108 of the third ventricle 110 with probe 102 (FIG. 2) to form an opening 206 in floor 108; and measuring a flow of cerebrospinal fluid (CSF) with a flow sensor 202 of probe 102. The ETV procedure can further include deploying a membrane eyelet 402 (FIGS. 4 and 5) into opening 206; and measuring a flow 714 (FIG. 7) of CSF through opening 206 with a flow sensor 412A of the membrane eyelet 402A.

More particularly, FIG. 1 is a cross-section view of a human cranium 100 during an endoscopic third ventriculostomy (ETV) procedure using an endoscopic third ventriculostomy probe 102 in one embodiment according to the present invention. Initially, a burr hole 104 is formed in the skull 106. Probe 102 is passed through burr hole 104, through the cerebral cortex and through the underlying white matter to a location adjacent the floor 108 of the third ventricle 110 as illustrated in FIG. 1. Probe 102 is then poked through floor 108 of third ventricle 110 to fenestrate floor 108 and the underlying membrane of Lillequist. (The drawings show a rounded end, but other end configurations suitable for piercing may be used.)

FIG. 2 is an enlarged view of the region II of FIG. 1 after fenestrating of floor 108 with probe 102. As shown in FIG. 2, probe 102 includes a shaft 201 and a flow sensor 202 mounted on or integral with shaft 201. Flow sensor 202 is adjacent to or forms a distal end 204 of probe 102. Flow sensor 202 can be a hot wire anemometer flow sensor as discussed further below in reference to FIG. 6.

As shown in FIG. 2, after poking through (fenestrating) floor 108, an opening 206, sometimes called fenestration, is formed in floor 108. Cerebrospinal fluid (CSF) from third ventricle 110 flows around probe 102 and through opening 206 into the interpeduncular cistern 208, thus relieving pressure from third ventricle 110. (The underlying membrane of Lillequist is also fenestrated.) Flow sensor 202 senses the flow of CSF.

In one embodiment, after the physician makes the poke with probe 102, probe 102 is left in place for a period of time, e.g., one minute, while flow sensor 202 measures the flow of CSF. In this manner, any flow of CSF measured by flow sensor 202 is flow of CSF through opening 206 and not movement of CSF relative to probe 102 due to motion of probe 102 itself. Stated another way, probe 102 is left in place for a period of time to avoid false positive CSF flow determinations.

FIG. 3 is an enlarged view of the region II of FIG. 1 at a later stage of the ETV procedure. Probe 102 is withdrawn slightly after the physician makes the poke with probe 102. More particularly, probe 102 is withdrawn out of opening 206.

By withdrawing probe 102, the amount of CSF flowing through opening 206 is increased. The greater the flow of CSF, the greater the reliably of the flow sensor 202 reading and actual correlation of the flow of CSF. Thus, by withdrawing probe 102 from opening 206, flow sensor 202 reading has an increased reliability of confirming the flow of CSF.

In the event that flow sensor 202 measures a flow of CSF, a determination is made that the ETV procedure is successful, i.e., that opening 206 was successfully formed. Accordingly, probe 102 is withdrawn from the patient.

Thus, by using probe 102, the ETV procedure is determined to be successful without the necessity of transferring the patient to a separate location for observation with a magnetic resonance imaging (MRI) device as in a conventional ETV procedure. This, in turn, minimizes the ETV procedure time as well as the expense and complexity of the ETV procedure.

Further, if flow sensor 202 readily does not detect a flow of CSF, thus indicating stasis of the CSF, i.e., the flow measurement is zero, a determination is made that a hole in floor 108 was not formed. In this event, the physician simply makes another poke using probe 102 to fenestrate floor 108 and the underlying membrane.

Thus, using probe 102, even if the initial attempt at fenestrating floor 108 fails, this failure is detected immediately. The probe poke is determined to be unsuccessful without the necessity of transferring the patient to a separate location for observation with a MRI device as in a conventional ETV procedure. Further, floor 108 can again be poked using the same probe 102 in contrast to having to repeatedly insert and withdraw a probe as in the prior art. This, in turn, minimizes the risk of infection and eliminates the possibility of causing damage to the cerebral cortex and the underlying white matter from repeated probe insertions.

FIG. 4 is an enlarged view of the region II of FIG. 1 at a later stage of the ETV procedure. Referring now to FIGS. 1 and 4 together, a membrane eyelet catheter 400 is passed through burr hole 104, through the cerebral cortex and through the underlying white matter. Membrane eyelet catheter 400 is inserted into opening 206.

Referring now to FIG. 4, membrane eyelet catheter 400 includes a membrane eyelet 402 mounted on a dilation balloon 404. Membrane eyelet 402 includes a waist section 406, a first anchor section 408, and a second anchor section 410. Further, membrane eyelet 402 includes a flow sensor 412 for measuring the flow of CSF through opening 206.

Membrane eyelet catheter 400 is inserted into opening 206 such that waist section 406 of membrane eyelet 402 is located in opening 206 and anchor sections 408 and 410 are located on either side (up or down in the view of FIG. 4) of floor 108.

FIG. 5 is an enlarged view of the region II of FIG. 1 at a later stage of the ETV procedure. Referring now to FIGS. 4 and 5 together, once positioned in opening 206 as described above, dilation balloon 404 is inflated to flare anchor section 408 and 410 from waist section 406. This sandwiches floor 108 between anchor sections 408 and 410 thus engaging membrane eyelet 402 to floor 108. Dilation balloon 404 is then deflated and withdrawn thus leaving membrane eyelet 402 engaged with floor 108.

Waist section 406 keeps opening 206 open. This minimizes the possibility of closure of opening 206 thus insuring the long-term success of the ETV procedure.

Flow sensor 412 is a wireless flow sensor such that flow sensor 412 can be remotely interrogated from outside the patient in one embodiment. Thus, the patency of opening 206 can be quickly and easily verified by using flow sensor 412 to determining that CSF is flowing through opening 206. Thus, using membrane eyelet 402, the patency of opening 206 is verified without the necessity of observing the patient with a MRI device as in a conventional ETV procedure. This, in turn, minimizes the complexity and expense associated with the ETV procedure.

Further, using membrane eyelet 402, the patency of opening 206 can be monitored continuously or frequently thus resulting in a rapid diagnosis of any closure of opening 206. By having rapid diagnosis, the complications associated with closure of opening 206 can be mitigated.

As discussed above and illustrate in FIG. 5, membrane eyelet 402 is radially expanded using dilation balloon 404. However, in another embodiment, a longitudinal compression of a mesh of juxtaposed fibers is used to radially expand membrane eyelet 402. Use of a mesh is well known to those of skill in the art and so is not discussed further.

In another embodiment, a membrane eyelet is self-expanding. In accordance with this embodiment, the membrane eyelet is constrained within a sheath (not shown). Retraction of the sheath exposes membrane eyelet, which self-expands. Use of a sheath to deploy a self-expanding device is well known to those of skill in the art and so is not discussed further.

FIG. 6 is a side view of distal end 204 of a probe 102A similar to probe 102 illustrated in FIGS. 1, 2 and 3. Referring now to FIG. 6, flow sensor 202 of probe 102A includes a heat source H, a first temperature sensor TS1 and a second temperature sensor TS2. Probe 102A includes an internal lumen (not shown) through which leads 602, 604, and 606 pass to heat source H, first temperature sensor TS1 and second temperature sensor TS2.

More particularly, first temperature sensor TS1 is located on one side (lower in the view of FIG. 6) of heat source H and second temperature sensor TS2 is located on the opposite side (upper in the view of FIG. 6) of heat source H.

Heat source H produces heat and thus heats temperature sensors TS1, TS2. The temperature on either side of heat source H is measured by temperature sensors TS1 and TS2.

More particularly, lead 602 is coupled to a power supply 610 that provides power to heat source H through lead 602. Further, leads 604 and 606 are coupled to a temperature measurement circuit 612, which receives signals from temperature sensors TS1 and TS2. Temperature measurement circuit 612 uses the signals from temperature sensors TS1 and TS2 to determine a temperature differential between temperature sensors TS1 and TS2.

If there is stasis of the CSF, then temperature sensors TS1 and TS2 measure the same temperature, and there is little or no temperature differential between temperature sensors TS1 and TS2.

However, if there is flow of CSF past probe 102A as indicated by arrow 614, then the temperature measured by temperature sensor TS1 is greater than the temperature measured by temperature sensor TS2. More particularly, as the CSF flows past temperature sensor TS2 and heat source H, the CSF becomes heated. Thus, the temperature of the CSF adjacent temperature sensor TS2 is less than the temperature of the CSF adjacent temperature sensor TS1 when there is a flow of CSF in the direction of arrow 614. By measuring this temperature differential between temperature sensors TS1 and TS2, a flow of CSF is detected.

In one embodiment, if the temperature differential between temperature sensors TS1 and TS2 is greater than or equal to a set point temperature differential, e.g., a 1 degree Fahrenheit temperature differential, a determination is made that there is a flow of CSF.

However, if the temperature differential between temperature sensors TS1 and TS2 is less than the set point temperature differential, a determination is made that there is not a flow of CSF, i.e., stasis.

In accordance with this embodiment, a determination is made as to whether there is or is not a flow of CSF, but the actual amount of CSF flow is not measured. However, in another embodiment, the temperature differential between temperature sensors TS1 and TS2 is calibrated to measure the actual amount of CSF flow.

FIG. 7 is an enlarged partial view of FIG. 5 of a membrane eyelet 402A engaged with floor 108 after withdrawal of dilation balloon 404 (FIG. 5). In FIG. 7, only the parts of anchor sections 408 and 410 to the left and right of the figure are illustrated for clarity.

Referring now to FIG. 7, a flow sensor 412A is a separate device that is coupled, e.g., welded, fused, clipped or otherwise joined, to waist section 406, first anchor section 408 and/or second anchor section 410 of membrane eyelet 402A.

Flow sensor 412A of membrane eyelet 402A includes a heat source HA, a first temperature sensor TS1A and a second temperature sensor TS2A. Further, flow sensor 412A includes a thermal circuit TCA for powering heat source HA, measuring a temperature differential between first temperature sensor TS1A and a second temperature sensor TS2A, and for receiving and transmitting signals to a remote interrogation device (not shown). Thermal circuit TCA is coupled to heat source HA opposite temperature sensors TS1A, TS2A or is integral (not shown) with heat source HA.

More particularly, first temperature sensor TS1A and second temperature sensor TS2A are arranged as ears protruding from and coupled to heat source HA. First temperature sensor TS1 A is spaced apart (down in the view of FIG. 7) from second temperature sensor TS2A. Thus, temperature sensor TS1A is located downstream of temperature sensor TS2A, i.e., the CSF flows past temperature sensor TS2A first and then flows past temperature sensor TS1A as indicated by the arrow 714.

Heat source HA produces heat and thus heats temperature sensors TS1A, TS2A. Flow sensor 412A is well suited for measuring oscillatory flow of CSF across flow sensor 412A.

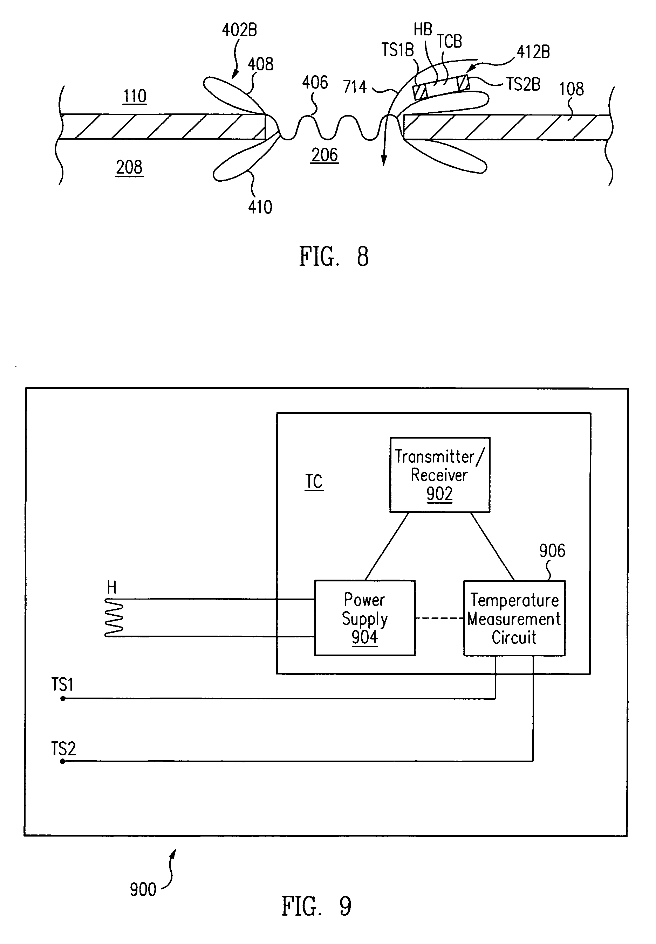

FIG. 8 is an enlarged partial view of FIG. 5 of membrane eyelet 402B engaged with floor 108 after withdrawal of dilation balloon 404 (FIG. 5) in another embodiment according to the present invention. In FIG. 8, only the parts of anchor sections 408 and 410 to the left and right of the figure are illustrated for clarity.

Referring now to FIG. 8, a flow sensor 412B is a separate device that is coupled, e.g., welded, fused, clipped or otherwise joined, to waist section 406, first anchor section 408 and/or second anchor section 410 of membrane eyelet 402B.

Flow sensor 412B of membrane eyelet 402B includes a heat source HB, a first temperature sensor TS1B and a second temperature sensor TS2B. Further, flow sensor 412B includes a thermal circuit TCB for powering heat source HB, measuring a temperature differential between first temperature sensor TS 1 B and a second temperature sensor TS2B, and for receiving and transmitting signals to a remote interrogation device (not shown). In accordance with this embodiment, thermal circuit TCB is integral with heat source HB.

More particularly, first temperature sensor TS1B is locate on one side of heat source HB and thermal circuit TCB and second temperature sensor TS2B is locate on the opposite side of heat source HB and thermal circuit TCB.

Heat source HB produces heat and thus heats temperature sensors TS1B, TS2B. The temperature on either side of heat source HB is measured by temperature sensors TS1B and TS2B. In accordance with this embodiment, temperature sensor TS1B is located downstream of temperature sensor TS2B, i.e., the CSF flows past temperature sensor TS2B first and then flows past temperature sensor TS1B as indicated by the arrow 714. Flow sensor 412B is well suited for measuring oscillatory flow of CSF across flow sensor 412B.

FIG. 9 is a circuit schematic diagram 900 of flow sensors 412A and 412B of FIGS. 7 and 8. Referring now to FIGS. 7, 8 and 9 together, thermal circuit TC (thermal circuits TCA and TCB of FIGS. 7 and 8 are generally referred to as thermal circuit TC in FIG. 9 for simplicity of discussion) includes an exemplary arrangement as follows: a transmitter/receiver 902, a power supply 904, e.g., a battery, and a temperature measurement circuit 906.

Transmitter/receiver 902, e.g., an antenna, receives a signal, e.g., an RF signal, from a remote interrogation device (not shown). Transmitter/receiver 902 is coupled to power supply 904. Power supply 904 converts the received signal into energy to power heat source H (heat sources HA and HB of FIGS. 7 and 8 are generally referred to as heat source H in FIG. 9 for simplicity of discussion). Heat source H is coupled to power supply 904. In one embodiment, power supply 904 is also coupled to and powers temperature measurement circuit 906.

Temperature measurement circuit 906 is coupled to and receives signals from temperature sensors TS1 and TS2 (temperature sensors TS1A, TS1B and TS2A, TS2B of FIGS. 7 and 8 are generally referred to as temperature sensors TS1 and TS2, respectively, in FIG. 9 for simplicity of discussion). Temperature measurement circuit 906 uses the signals from temperature sensors TS1 and TS2 to determine a temperature differential between temperature sensors TS1 and TS2.

If there is stasis of the CSF, then temperature sensors TS1 and TS2 measure the same temperature, and there is little or no temperature differential between temperature sensors TS1 and TS2.

However, if there is flow of CSF, then the temperature measured by temperature sensor TS1 is greater than the temperature measured by temperature sensor TS2. More particularly, as the CSF flows past temperature sensor TS2 and heat source H, the CSF becomes heated. Thus, the temperature of the CSF adjacent temperature sensor TS2 is less than the temperature of the CSF adjacent temperature sensor TS1 when there is a flow of CSF in the direction of arrow 714. By measuring this temperature differential between temperature sensors TS1 and TS2, a flow of CSF is detected.

In one embodiment, if the temperature differential between temperature sensors TS1 and TS2 is greater than or equal to a set point temperature differential, e.g., a 1 degree Fahrenheit temperature differential, a determination is made that there is a flow of CSF through opening 206.

However, if the temperature differential between temperature sensors TS1 and TS2 is less than the set point temperature differential, a determination is made that there is not a flow of CSF, i.e., stasis. In accordance with this embodiment, a determination is made as to whether there is or is not a flow of CSF, but the actual amount of CSF flow is not measured. However, in another embodiment, the temperature differential between temperature sensors TS1 and TS2 is calibrated to measure the actual amount of CSF flow.

Temperature measurement circuit 906 is coupled to transmitter/receiver 902. Temperature measurement circuit 906 sends a signal representing the measured temperature differential between temperature sensors TS1, TS2 to transmitter/receiver 902. Transmitter/receiver 902 transmits this signal to the remote interrogation device. In the above matter, the flow of CSF through opening 206, and thus the patency of opening 206, is noninvasively measured and recorded. Although two temperature sensors TS1, TS2 are set forth, a flow sensor having three temperature sensors can be used, e.g., to measure oscillatory flow of CSF across the flow sensor.

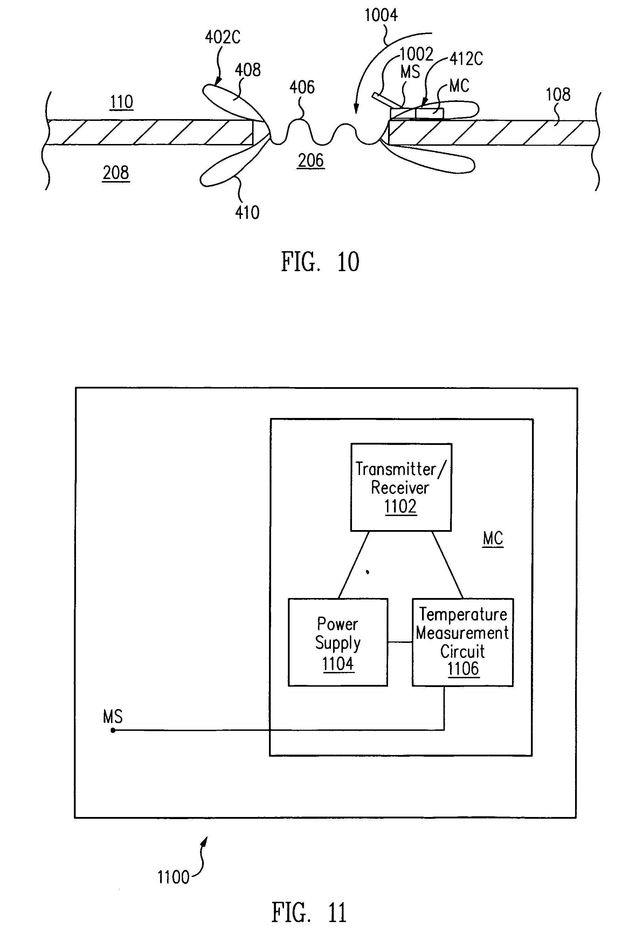

FIG. 10 is an enlarged partial view of FIG. 5 of membrane eyelet 402C engaged with floor 108 after withdrawal of dilation balloon 404 (FIG. 5) in another embodiment according to the present invention. In FIG. 10, only the parts of anchor sections 408 and 410 to the left and right of the figure are illustrated for clarity.

Referring now to FIG. 10, a flow sensor 412C is a separate device that is coupled, e.g., welded, fused, clipped or otherwise joined, to waist section 406, first anchor section 408 and/or second anchor section 410 of membrane eyelet 402C.

Flow sensor 412C of membrane eyelet 402C includes a miniature movable element 1002 coupled to a motion sensor MS of flow sensor 412C. Motion sensor MS is for detecting the motion of miniature movable element 1002.

Further, flow sensor 412C includes a motion circuit MC for receiving a signal from motion sensor MS and for receiving and transmitting signals to a remote interrogation device (not shown). Motion circuit MC is coupled to motion sensor MS or is integral with motion sensor MS (not shown).

More particularly, miniature movable element 1002, e.g., a lever or pinwheel, is located within the flow of CSF as indicated by arrow 1004. As is well known to those of skill in the art, CSF flow pulsates. This pulsation in the CSF flow moves miniature movable element 1002. This movement of miniature movable element 1002 is detected by motion sensor MS.

Illustratively, a change in capacitance resulting from the movement of miniature movable element 1002 is measured by motion sensor MS. As another example, miniature movable element 1002 is a pinwheel and motion sensor MS is a piezo-electric crystal, which produces a signal upon rotation of the pinwheel.

FIG. 11 is a circuit schematic diagram 1100 of flow sensor 412C of FIG. 10. Referring now to FIGS. 10 and 11 together, motion circuit MC includes a transmitter/receiver 1102, a power supply 1104, and a motion measurement circuit 1106.

Transmitter/receiver 1102, e.g., an antenna, receives a signal, e.g., an RF signal, from a remote interrogation device (not shown). Transmitter/receiver 1102 is coupled to power supply 1104, which converts the received signal into energy to power motion measurement circuit 1106, which is coupled to power supply 1104.

However, in another embodiment (not shown), motion circuit MC does not include power supply 1104. In accordance with this embodiment, motion of miniature moveable element 1002 is translated into power for the operation of flow sensor 412C.

Motion measurement circuit 1106 is coupled to and receives a signal from motion sensor MS. Motion measurement circuit 1106 uses the signal from motion sensor MS to determine whether there is a flow of CSF.

If there is stasis of the CSF, then miniature moveable element 1002 does not move and so no motion of miniature moveable element 1002 is detected by motion sensor MS. In this event, there is no variation in the signal, e.g., no signal, from motion sensor MS to motion measurement circuit 1106.

However, if there is flow of CSF, then miniature moveable element 1002 does move and so motion of miniature moveable element 1002 is detected by motion sensor MS. In this event, there is variation in the signal from motion sensor MS to motion measurement circuit 1106.

In one embodiment, if there is variation in the signal from motion sensor MS to motion measurement circuit 1106, a determination is made that there is a flow of CSF. Conversely, if there is not a variation in the signal, e.g., no signal, from motion sensor MS to motion measurement circuit 1106, a determination is made that there is not a flow of the CSF., i.e., stasis. In accordance with this embodiment, a determination is made as to whether there is or is not a flow of CSF, but the actual amount of CSF flow is not measured. However, in another embodiment, the amount of motion of miniature moveable element 1002 is calibrated to measure the actual amount of CSF flow.

Motion measurement circuit 1106 outputs a signal representing motion or lack of motion of miniature moveable element 1002 to transmitter/receiver 1102. Transmitter/receiver 1102 transmits this signal to the remote interrogation device. In the above matter, the flow of CSF through opening 206, and thus the patency of opening 206, is noninvasively measured and recorded.

This disclosure provides exemplary embodiments of the present invention. The scope of the present invention is not limited by these exemplary embodiments. Numerous variations, whether explicitly provided for by the specification or implied by the specification or not, such as variations in structure, dimension, type of material and manufacturing process may be implemented by one of skill in the art in view of this disclosure. For example, it is possible to combine different embodiments of the flow sensor in a single membrane eyelet or ETV probe.

According to a method aspect of the present invention, a burr hole is formed in a human skull, an endoscopic third ventriculostomy (ETV) probe is passed through said burr hole, the floor of the third ventricle is fenestrated with said ETV probe, and a flow of cerebrospinal fluid (CSF) is measured with a flow sensor of said ETV probe. Said flow indicates whether said fenestrating was successful or not.

Preferably, the flow of CSF measured during said measuring allows to determine whether said floor was fenestrated during said fenestrating or not. When no flow of CSF is measured during said measuring it is determined that said floor was not fenestrated during said fenestrating. In this event, said fenestration is repeated.

According to another method aspect of the present invention, the floor of the third ventricle is fenestrated with an endoscopic third ventriculostomy probe to form an opening in said floor, and a membrane eyelet is deployed into said opening. Further to this, a flow of cerebrospinal fluid (CSF) through said opening is measured with a flow sensor of said membrane eyelet. Optionally, said measuring of a flow also comprises determining a temperature differential between a first temperature sensor and a second temperature sensor of said flow sensor. Preferably, measuring a flow comprises also determining a movement of a miniature moveable element of said flow sensor.

According to another embodiment, said deploying of the membrane eyelet comprises sandwiching said floor between a first anchor section and a second anchor section of said membrane eyelet. Further to this, the patency of said opening can be maintained with a waist section of said membrane eyelet.

The deploying aof the membrane eyelet may comprises radially expanding said membrane eyelet. Preferably, said radially expanding is performed with a dilation balloon.

## Claims

1. An endoscopic third ventriculostomy (ETV) probe (102) comprising:
a shaft (201); and
a flow sensor (202) mounted on said shaft (201).

2. The ETV probe (102) of claim 1 wherein said probe (102) is suitable for forming an opening in the floor of the third ventricle.

3. The ETV probe (102) of claim 2 wherein said flow sensor (202) is suitable for measuring a flow of cerebrospinal fluid (CSF) through said opening.

4. The ETV probe (102) according to any of the preceding claims, wherein said flow sensor (202) comprises a hot wire anemometer flow sensor.

5. The ETV probe (102) according to any of the preceding claims, wherein said flow sensor (202) comprises a heat source (H), a first temperature sensor (TS 1) and a second temperature sensor (TS2).

6. The ETV probe (102) of claim 5, wherein said first temperature sensor (TS 1) and said second temperature sensor (TS2) are on opposite sides of said heat source (H).

7. The ETV probe (102) of claim 5 or 6, wherein a temperature differential between a temperature measured by said first temperature sensor (TS 1) and a temperature measured by said second temperature sensor (TS2) is equal to or greater than a set point temperature differential.

8. The ETV probe (102) of claim 7, wherein said temperature differential indicates a flow of cerebrospinal fluid (CSF) around said ETV probe (102).

9. The ETV probe (102) of claim 5 or 6, wherein a temperature differential between a temperature measured by said first temperature sensor (TS1) and a temperature measured by said second temperature sensor (TS2) is less than a set point temperature differential.

10. The ETV probe (102) of claim 9 said temperature differential indicates stasis of cerebrospinal fluid (CSF) around said ETV probe (102).

11. A membrane eyelet (402) comprising:
a waist section (406);
a first anchor section (408) coupled to said waist section (406);
a second anchor section (410) coupled to said waist section (406); and
a flow sensor (412) coupled to said waist section (406), said first anchor section (408) and/or said second anchor section (410).

12. The membrane eyelet (402) of claim 11, wherein said flow sensor (412) comprises a hot wire anemometer flow sensor.

13. The membrane eyelet (402) of claim 11, wherein said flow sensor (412) comprises a MEMS flow sensor.

14. The membrane eyelet (402A, 402B) according to any of claims 11 to 13, wherein said flow sensor (412A, 412B) comprises:
a heat source (HA, HB);
a first temperature sensor (TS1A, TS1B) coupled to said heat source (HA, HB); and
a second temperature sensor (TS2A, TS2B) coupled to said heat source (HA, HB).

15. The membrane eyelet (402A, 402B) of claim 14, wherein said flow sensor (412A, 412B) further comprises a thermal circuit (TCA, TCB) for measuring a temperature differential between said first temperature sensor (TS1A, TS1B) and said second temperature sensor (TS2A, TS2B).

16. The membrane eyelet (412A, 412B) of claim 15, wherein said thermal circuit (TCA, TCB) comprises:
a transmitter/receiver (902);
a power supply (904) coupled to said transmitter/receiver (902); and
a temperature measurement circuit (906) coupled to said transmitter/receiver (902).

17. The membrane eyelet (402A, 402B) of claim 16, wherein said power supply (904) is coupled to said heat source (HA, HB).

18. The membrane eyelet (402A, 402B) of claim 16 or 17, wherein said temperature measurement circuit (906) is coupled to said first temperature sensor (TS1A, TS1B) and said second temperature circuit (TS1A, TS1B).

19. The membrane eyelet (402C) according to any of claims 11 to 18, wherein said flow sensor (412C) comprises:
a miniature moveable element (1002); and
a motion sensor (MS) coupled to said miniature moveable element (1002).

20. The membrane eyelet (412C) of claim 19, wherein said flow sensor (412C) further comprises a motion circuit (MC) coupled to said motion sensor (MS).

21. The membrane eyelet (412C) of claim 20, wherein said motion circuit (MC) comprises:
a transmitter/receiver (1102); and
a motion measurement circuit (1106) coupled to said transmitter/receiver (1102).

22. The membrane eyelet (412C) of claim 21, wherein said motion circuit (MC) further comprises a power supply (1104) coupled to said motion measurement circuit (1106).

23. The membrane eyelet (412C) of claim 21 or 22, wherein said motion measurement circuit (1106) is coupled to said motion sensor (MS).
